# FASCICULE DE BREVET EUROPEEN

(11) **EP 1 137 369 B1**
(45) Date de publication et mention de la délivrance du brevet: **24.11.2004**
(21) Numéro de dépôt: 99915823.1
(22) Date de dépôt: 23.04.1999
(51) Int. Cl.: A61B 17/22

(54) **FILTRE SANGUIN TEMPORAIRE**
TEMPORÄRER BLUTFILTER
TEMPORARY BLOOD FILTER

(43) Date de publication de la demande: 04.10.2001
(73) Titulaire: Despalle de Bearn, Olivier, 38260 Marcilloles (FR)
(72) Inventeur: Despalle de Bearn, Olivier, 38260 Marcilloles (FR)
(74) Mandataire: Plucker, Guy
(86) Numéro de dépôt international: PCT/FR1999/000967
(87) Numéro de publication internationale: WO 2000/064356

(56) Documents cités:
- EP-A- 0 820 729
- WO-A-98/39053
- DE-A- 19 715 890
- DE-U- 8 707 515
- FR-A- 2 768 326

## Description

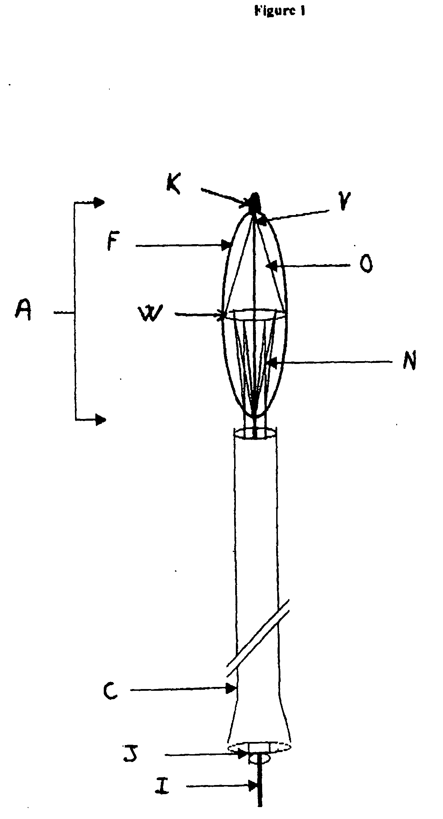

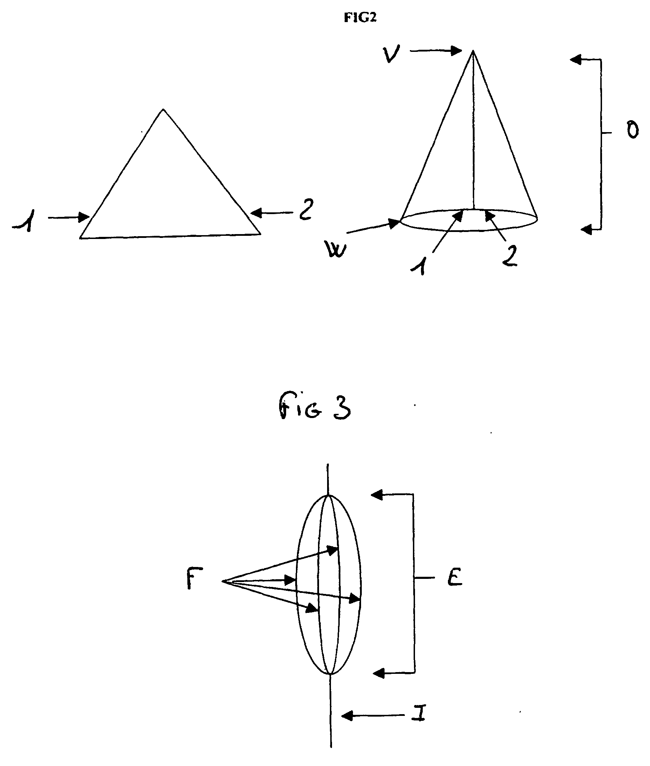

La présente invention conceme un filtre temporaire en forme de cône (O) monté, serti, à l'extrémité distale sous l'âme souple d'un guide métallique (I), qui peut avoir différentes longueurs et diamètres (fig 1). Le guide (I) est inséré dans un tube métallique (J) et l'extrémité distale de ce tube métallique est découpée en couronne de plus ou moins quatre branches (N) qui sont fixées à la base du cône (O), le tout étant inséré dans un cathéter guide porteur (C) qui assure l'ouverture et la fermeture du système.

Les systèmes existants sont soit occlusifs par ballonnet soit non occlusifs par filet mais ne peuvent filtrer des particules inférieures à 200 microns et ne peuvent se refermer dans un cathéter guide porteur (C) pour le fermer.

Le document français FR 2 768 326 divulgue un système de filtre pour piéger les particules susceptibles de se détacher de la paroi des vaisseaux lors d'une intervention.
Ce système comprend un filet en forme de cône monté sur l'extrémité distale d'un guide métallique par l'intermédiaire d'un anneau souple et l'ensemble étant inséré dans un cathéter guide porteur. Le filet est déployé en poussant le guide métallique et est escamoté en tirant sur le même guide.
Cependant dans ce genre de dispositif, le filet n'est soutenu que par deux fils de Nitinol.

La présente invention sert à protéger les voies cérébrales, principalement dans les carotides ou autres, des particules pouvant se détacher de la lumière inteme d'une artère ou d'une veine, traitée par angioplastie. ou autre en aval du filtre (A).
Le présent dispositif est introduit par voie endoluminale à travers un introducteur et retiré au travers du même introducteur.
Ce dispositif permet, une fois le filtre (A) en place, de monter tout système de dilatation et de contrôle.

L'invention est définie à la revendication 1. Les autres revendications donnent des modes de réalisation.

En référence aux dessins. le dispositif comporte en effet selon une première caractéristique, un filtre en forme de cône (O) pouvant filtrer des particules de plus ou moins 48 microns.

Etant réalisé dans un textile ou autres, ce cône (O) est réalisé à partir d'un triangle de textile à plat qui est mis en forme de cône (O) en joignant les bords (1) et (2) par une couture, par une soudure à ultrason ou par collage.

La deuxième caractéristique est que le guide (I), selon des modes particuliers de réalisation qui consiste en les étapes suivantes : (I) est coupé en deux ou plus dans le sens de la longueur à l'extrémité du guide (I) sur quelques centimètres afin de réaliser un logement en forme « cage » ovale (E). Cette forme de cage est obtenue par un traitement thermique. Les brins (F) de la « cage » ovale (E) ont subi un traitement particulier afin de les rendre radiopaques.

A l'intérieur de la « cage » ovale (E) est fixée la pointe (V) du cône (O) à l'extrémité distale de la « cage » ovale (E) par soudure, colle ou couture.

La base (w) de ce même cône (O) est fixée à l'intérieur des brins (F) de la « cage » ovale (E) obtenus grâce à la découpe laser, a une hauteur suffisante pour permettre le déploiement complet du cône (O).
Une bague radiopaque (K) en forme d'ogive est fixée à l'extrémité distale de la « cage » ovale (E).
Le guide (I) est inséré dans un tube métallique (J) dont l'extrémité distale est découpée en couronne à plus ou moins quatre branches (N). Sur ces branches (N) est fixée la base du cône (O).
Le tout est inséré dans un cathéter guide porteur (C) qui assure l'ouverture et la fermeture du système.

Le dispositif selon l'invention est particulièrement destiné à un usage médical. La réalisation de ce dispositif peut être réalisée par laser.

## Revendications

1. Filtre (A) pour protéger les voies cérébrales des particules pouvant être détachées de la lumière d'une artère ou d'une veine traitée par angioplastie en aval dudit filtre (A), introduit à travers un introducteur et pouvant être retiré après une intervention endoluminale au travers dudit introducteur, ledit filtre comprenant un guide métallique (1) inséré dans un tube métallique (J) et un cône filtrant (O) dont la pointe est fixée près de l'extrémité du guide (I), **caractérisé en ce que** l'extrémité distale du tube métallique est découpée en une couronne à plus ou moins quatre branches (N), le cône (O) filtrant est réalisé en textile et filtre des particules de plus ou moins 48 micromètres, la base du cône (O) étant fixée sur les branches de la couronne.

2. Filtre selon la revendication 1, **caractérisé en ce que** le cône (O) est réalisé à partir d'un triangle de textile plat, mis en forme en joignant les bords (1) et (2) par une couture, par une soudure à ultrason ou par collage.

3. Filtre selon la revendication 1 ou 2 **caractérisé en ce que** le guide est coupé au laser en deux ou plus dans le sens de la longueur à quelques millimètres de l'extrémité distale du guide (I) afin de réaliser un cage de forme d'ovale (E), cette cage (E) étant obtenue par un traitement thermique ou autre et dans la partie haute de la cage en forme d'ovale (E) du guide (I) étant fixée au sommet (V) du cône (O) par soudure, collage oü sertissage, la base du cône étant fixée à l'intérieur des brins (F) de la cage qui sont obtenus grâce à la découpe laser, à une hauteur suffisante pour permettre le déploiement complet du filtre (A) lors de l'ouverture du dispositif.

4. Filtre selon la revendication 3 **caractérisé en ce que** les brins (F) de la cage ovale (E) ont subi un traitement particulier afin de les rendre radiopaques et de faciliter le repérage du dispositif sous radioscopie.

5. Filtre selon l'une quelconque des revendications 3 ou 4, **caractérisé en ce que** la base (W) du cône (O) est fixé à l'intérieur des brins (F) de la cage ovale (E) obtenus grâce à la découpe laser, a une hauteur suffisante pour permettre le déploiement complet du cône (O), une bague radiopaque (K) en forme d'ogive étant fixée à l'extrémité distale de la cage ovale (E) afin de rendre le système atraumatique et sur ces branches (N) est fixée la base du cône (O), le tout étant inséré dans un cathéter guide porteur (C) qui assure l'ouverture et la fermeture du système.

6. Filtre selon l'une quelconque des revendications 1 à 5, **caractérisé en ce qu'**il est découpé ou soudé à l'extrémité d'un tube métallique (J) à mémoire de forme, au moins deux lamelles (H) métalliques à l'extrémité desquelles est fixée la base (W) du cône (O) permettant le déploiement complet du filtre (A), à l'intérieur du tube métallique (J) est introduit le guide (I) métallique dont l'extrémité distale droite est fixée au sommet (V) du cône (O) par une bague radiopaque (K) et la fermeture et l'ouverture étant réalisées grâce au cathéter guide porteur (C)

7. Filtre selon l'une quelconque des revendications 1 à 5, **caractérisé en ce qu'**il est découpé ou soudé à l'extrémité d'un tube métallique (J) à mémoire de forme, au moins deux lamelles (H) métalliques à l'extrémité desquelles est fixée la base (W) du cône (O) permettant le déploiement complet du filtre (A), à l'intérieur du tube métallique (J) est introduit le guide (I) métallique, dont l'extrémité distale est en forme de spirale et l'extrémité du guide (I) est fixée au sommet M du cône (O) par une bague radiopaque (K) et la fermeture et l'ouverture étant réalisées grâce au cathéter guide porteur (C).

8. Filtre selon l'une quelconque des revendications 1 à 5, **caractérisé en ce qu'**il est découpé ou soudé à l'extrémité d'un tube métallique (J) à mémoire de forme, au moins deux lamelles (H) métalliques à l'extrémité desquelles est fixée la base (W) du cône (O) permettant le déploiement complet du filtre (A), à l'intérieur du tube métallique (J) est introduit le guide (I) métallique dont l'extrémité distale est découpée au moins en deux, dans le sens de la longueur, sur quelques centimètres et est en forme de tulipe et fixée au sommet (V) du cône (O) par une bague radiopaque, la fermeture et l'ouverture étant réalisées grâce au cathéter guide porteur (C).

## Claims

1. Filter (A) for protecting the cerebral ducts from particles that may become detached from the lumen of an artery or vein treated by angioplasty downstream of said filter (A), the latter being introduced via an introducer and being able to be withdrawn via said introducer after an intraluminal intervention, said filter comprising a metal guide (I) inserted into a metal tube (J), and a filtering cone (O) whose tip is fixed near the end of the guide (I), **characterized in that** the distal end of the metal tube is cut into a crown of approximately four branches (N), the filtering cone (O) is made of textile and filters particles of approximately 48 micrometres, the base of the cone (O) being fixed to the branches of the crown.

2. Filter according to Claim 1, **characterized in that** the cone (O) is made from a flat textile triangle, shaped by joining the edges (1) and (2) by stitching, ultrasonic welding or adhesive bonding.

3. Filter according to Claim 1 or 2, **characterized in that** the guide is cut in two or more by laser in the direction of the length, several millimetres from the distal end of the guide (I), in order to form an oval-shaped cage (E), this cage (E) being obtained by thermal or other treatment and, in the upper part of the oval-shaped cage (E) of the guide (I), being fixed to the top (V) of the cone (O) by welding, adhesive bonding or crimping, the base of the cone being fixed to the inside of the strands (F) of the cage which are obtained by the laser cutting, at a height sufficient to permit complete deployment of the filter (A) upon opening of the device.

4. Filter according to Claim 3, **characterized in that** the strands (F) of the oval cage (E) have undergone a specific treatment in order to make them radiopaque and to make it easier to locate the device by radioscopy.

5. Filter according to either of Claims 3 and 4, **characterized in that** the base (W) of the cone (O) is fixed to the inside of the strands (F) of the oval cage (E) which are obtained by the laser cutting, at a height sufficient to permit complete deployment of the cone (0), a radiopaque ring (K) of bulb shape being fixed to the distal end of the oval cage (E) in order to make the system atraumatic, and the base of the cone (0) is fixed on these branches (N), the whole assembly being inserted into a supporting guide catheter (C) which ensures the opening and closure of the system.

6. Filter according to any one of Claims 1 to 5, **characterized in that** it is cut or welded at the end of a metal tube (J) of shape-memory metal, at least two metal leaves (H) at whose ends the base (W) of the cone (O) is fixed permitting complete deployment of the filter (A), the metal guide (I) is introduced inside the metal tube (J) and its straight distal end is fixed to the top (V) of the cone (0) by a radiopaque ring (K), and closure and opening are obtained by virtue of the supporting guide catheter (C).

7. Filter according to any one of Claims 1 to 5, **characterized in that** it is cut or welded at the end of a metal tube (J) of shape-memory metal, at least two metal leaves (H) at whose ends the base (W) of the cone (O) is fixed permitting complete deployment of the filter (A), the metal guide (I) is introduced inside the metal tube (J) and its distal end is in the shape of a spiral, and the end of the guide (I) is fixed to the top (V) of the cone (O) by a radiopaque ring (K), and closure and opening are obtained by virtue of the supporting guide catheter (C).

8. Filter according to any one of Claims 1 to 5, **characterized in that** it is cut or welded at the end of a metal tube (J) of shape-memory metal, at least two metal leaves (H) at whose ends the base (W) of the cone (O) is fixed permitting complete deployment of the filter (A), the metal guide (I) is introduced inside the metal tube (J) and its distal end is cut at least in two, in the direction of the length, along several centimetres, and has a tulip shape and is fixed to the top (V) of the cone (O) by a radiopaque ring, and closure and opening are obtained by virtue of the supporting guide catheter (C).

## Patentansprüche

1. Filter (A) zum Schutz zerebraler Kanäle vor Partikeln, die sich von dem Lumen einer Arterie oder einer Vene, die durch Angioplastie behandelt wird, stromabwärts des Filters (A), das durch einen Einführer eingeführt wird und nach einem endoluminalen Eingriff durch den Einführer wieder herausgezogen werden kann, ablösen können, wobei das Filter eine metallische Führung (I), die in eine metallische Röhre (J) eingefügt ist, und einen Filterkegel (O), dessen Spitze in der Nähe des Endes der Führung (I) befestigt ist, umfasst, **dadurch gekennzeichnet, dass** das distale Ende der metallischen Röhre kranzförmig in mehr oder weniger als vier Zweige (N) geschnitten ist, wobei der Filterkegel (O) aus Textil hergestellt ist und Partikel größer oder kleiner als 48 Mikrometer filtert, wobei die Basis des Kegels (O) auf den Zweigen des Kranzes befestigt ist.

2. Filter nach Anspruch 1, **dadurch gekennzeichnet, dass** der Kegel (O) ausgehend von einem flachen Textildreieck hergestellt ist, das durch Verbinden der Ränder (1) und (2) durch eine Naht, durch eine Ultraschallschweißung oder durch Kleben geformt wird.

3. Filter nach Anspruch 1 oder 2, **dadurch gekennzeichnet, dass** die Führung mit dem Laser zweimal oder mehr in Längsrichtung in einigen Millimetern vom distalen Ende der Führung (I) geschnitten ist, um einen Käfig (E) in ovaler Form herzustellen, wobei der Käfig (E) durch eine Wärmebehandlung oder anders erzielt wird und in dem oberen Teil des Käfigs (E) mit ovaler Form der Führung (I) an der Spitze (V) des Kegels (O) durch Schweißen, Kleben oder Falzen befestigt ist, wobei die Basis des Kegels im Inneren der Stränge (F) des Käfigs, die dank des Laserschnitts erzielt werden, in einer ausreichenden Höhe befestigt wird, um das komplette Auffalten des Filters (A) beim Öffnen der Vorrichtung zu erlauben.

4. Filter nach Anspruch 3, **dadurch gekennzeichnet, dass** die Stränge (F) des ovalen Käfigs (E) eine besondere Behandlung erhalten haben, um sie röntgenstrahlenundurchlässig zu machen und das Orten der Vorrichtung unter Radioskopie zu erleichtern.

5. Filter nach einem der Ansprüche 3 oder 4, **dadurch gekennzeichnet, dass** die Basis (W) des Kegels (O) im Inneren der Stränge (F) des ovalen Käfigs (E), die durch Laserschnitt erzielt werden, in einer ausreichenden Höhe befestigt ist, um das komplette Auffalten des Kegels (O) zu erlauben, wobei ein röntgenstrahlenundurchlässiger Ring (K) in Spitzbogenform am distalen Ende des ovalen Käfigs (E) befestigt ist, um das System atraumatisch zu machen, und auf diesen Zweigen (N) die Basis des Kegels (O) befestigt ist, wobei das Ganze in einen Trägerführungskatheter (C) eingeführt wird, der das Öffnen und das Schließen des Systems sicherstellt.

6. Filter nach einem der Ansprüche 1 bis 5, **dadurch gekennzeichnet, dass** am Ende einer Metallröhre (J) mit Formgedächtnis mindestens zwei metallische Lamellen (H) ausgeschnitten oder geschweißt sind, an deren Ende die Basis (W) des Kegels (O) befestigt ist, welche das komplette Auffalten des Filters (A) erlaubt, im Inneren der metallischen Röhre (J) die metallische Führung (I) eingeführt ist, deren distales gerades Ende an der Spitze (V) des Kegels (0) durch einen röntgenstrahlenundurchlässigen Ring (K) befestigt ist, und das Schließen und das Öffnen dank des Trägerführungskatheters (C) durchgeführt werden.

7. Filter nach einem der Ansprüche 1 bis 5, **dadurch gekennzeichnet, dass** am Ende einer metallischen Röhre (J) mit Formgedächtnis mindestens zwei metallische Lamellen (H) ausgeschnitten oder geschweißt sind, an deren Ende die Basis (W) des Kegels (O) befestigt ist, die das komplette Auffalten des Filters (A) erlaubt, im Inneren der metallischen Röhre (J) die metallische Führung (I) eingeführt ist, deren distales Ende Spiralform hat, und das Ende der Führung (I) auf der Spitze (V) des Kegels (O) durch einen röntgenstrahlenundurchlässigen Ring (K) befestigt ist, und das Öffnen und das Schließen dank des Trägerführungskatheters (C) durchgeführt werden.

8. Filter nach einem der Ansprüche 1 bis 5, **dadurch gekennzeichnet, dass** am Ende einer metallischen Röhre (J) mit Formgedächtnis mindestens zwei metallische Lamellen (H) ausgeschnitten oder geschweißt sind, an deren Ende die Basis (W) des Kegels (O) befestigt ist, die das komplette Auffalten des Filters (A) erlaubt, in das Innere der metallischen Röhre (J) die metallische Führung (I) eingeführt ist, deren distales Ende in die Längsrichtung über einige Zentimeter mindestens in zwei ausgeschnitten ist und Tulpenform hat und auf der Spitze (V) des Kegels (O) durch einen röntgenstrahlenundurchlässigen Ring befestigt ist, wobei das Schließen und das Öffnen dank des Trägerführungskatheters (C) durchgeführt werden.
